# EUROPEAN PATENT APPLICATION

(11) **EP 4 524 975 A1**
(43) Date of publication of application: **19.03.2025**
(21) Application number: 23803290.8
(22) Date of filing: 05.04.2023
(51) Int. Cl.: G16B 40/20, C07K 16/00, C12N 15/13, G01N 33/15, G16H 20/10

(54) **PHARMACEUTICAL ASSISTANCE DEVICE, METHOD OF OPERATING PHARMACEUTICAL ASSISTANCE DEVICE, AND PROGRAM FOR OPERATING PHARMACEUTICAL ASSISTANCE DEVICE**

(30) Priority: 09.05.2022 JP 2022077238
(71) Applicant: FUJIFILM Corporation, Tokyo 106-8620 (JP)
(72) Inventor: LI, Yuanzhong, Ashigarakami-gun, Kanagawa 258-8577 (JP)
(74) Representative: Hoffmann Eitle
(86) International application number: PCT/JP2023/014062
(87) International publication number: WO 2023/218807

(57) **Abstract**

A pharmaceutical support device includes a processor, in which the processor is configured to: acquire protein information related to a protein contained in a biopharmaceutical as a preservation target and including site information related to a plurality of sites constituting the protein, prescription information related to a prescription of a candidate preservation solution that is a candidate for a preservation solution for the biopharmaceutical as the preservation target, and measurement data indicating preservation stability of the candidate preservation solution measured by an actual test; derive, based on the protein information and the prescription information, a first feature amount related to the preservation stability of the candidate preservation solution for an entire protein as a target and a second feature amount related to the preservation stability of the candidate preservation solution for each of the plurality of sites as a target; and input the measurement data, the first feature amount, and the second feature amount to a machine learning model and output a score indicating the preservation stability of the candidate preservation solution from the machine learning model.

## Description

### BACKGROUND OF THE INVENTION

### 1. Field of the Invention

The technology of the present disclosure relates to a pharmaceutical support device, an operation method of a pharmaceutical support device, and an operation program of a pharmaceutical support device.

### 2. Description of the Related Art

In recent years, biopharmaceuticals have attracted attention due to high drug efficacy and low side effects. The biopharmaceutical has, for example, a protein, such as interferon or an antibody, as an active ingredient. The biopharmaceutical is preserved in a preservation solution. It is important to prescribe a preservation solution (also referred to as formulation prescription) suitable for the biopharmaceutical in order to stably maintain the quality of the biopharmaceutical.

The preservation solution includes a buffer solution, an additive, and a surfactant. The prescription of the preservation solution is, for example, the type and concentration of each of the buffer solution, the additive, and the surfactant and a hydrogen ion exponent (potential of hydrogen (pH) value) of the preservation solution.

In the related art, in a case where the prescription of the preservation solution is determined, a plurality of types of preservation solutions are prepared, for example, while changing a combination of the buffer solution, the additive, and the surfactant, and the preservation stability of each preservation solution is confirmed by an actual test. However, it takes a lot of time and effort to perform this operation. Therefore, for example, as in WO2021/041384A and <Theresa K. Cloutier, etc., Machine Learning Models of Antibody-Excipient Preferential Interactions for Use in Computational Formulation Design, Mol. Pharmaceutics, 17, 9, 3589-3599, 2020.> (hereinafter, referred to as Non-Patent Document), a technology has been proposed that predicts preservation stability of a preservation solution based on information of a protein in a biopharmaceutical or information of an additive in the preservation solution, using a molecular dynamics (MD) method or a machine learning model, without performing any test.

### SUMMARY OF THE INVENTION

In the technology disclosed in WO2021/041384A and Non-Patent Document, the preservation stability of the preservation solution is not confirmed by the actual test. In addition, in the technology disclosed in WO2021/041384A and Non-Patent Document, the preservation stability of each of the plurality of sites constituting the protein has not been analyzed in detail. Therefore, there is a risk that a prediction result that is far from the original preservation stability may be obtained.

An embodiment according to the technology of the present disclosure provides a pharmaceutical support device, an operation method of a pharmaceutical support device, and an operation program of a pharmaceutical support device that can improve prediction accuracy of preservation stability of a preservation solution.

According to the present disclosure, there is provided a pharmaceutical support device comprising a processor, in which the processor is configured to: acquire protein information related to a protein contained in a biopharmaceutical as a preservation target and including site information related to a plurality of sites constituting the protein, prescription information related to a prescription of a candidate preservation solution that is a candidate for a preservation solution for the biopharmaceutical as the preservation target, and measurement data indicating preservation stability of the candidate preservation solution measured by an actual test; derive, based on the protein information and the prescription information, a first feature amount related to the preservation stability of the candidate preservation solution for an entire protein as a target and a second feature amount related to the preservation stability of the candidate preservation solution for each of the plurality of sites as a target; and input the measurement data, the first feature amount, and the second feature amount to a machine learning model and output a score indicating the preservation stability of the candidate preservation solution from the machine learning model.

It is preferable that the protein information includes at least one of an amino acid sequence of the protein or a three-dimensional structure of the protein.

It is preferable that the prescription information includes a type and concentration of each of a buffer solution, an additive, and a surfactant contained in the candidate preservation solution and a hydrogen ion exponent of the candidate preservation solution.

It is preferable that the processor is configured to derive the first feature amount and the second feature amount using a molecular dynamics method.

It is preferable that the first feature amount includes at least one of a solvent accessible surface area, a spatial aggregation propensity, or a spatial charge map of the entire protein, and the second feature amount includes at least one of a solvent accessible surface area, a spatial aggregation propensity, or a spatial charge map of the sites constituting the protein.

It is preferable that the first feature amount includes an indicator indicating compatibility between the entire protein and an additive contained in the candidate preservation solution, and the second feature amount includes an indicator indicating compatibility between the sites constituting the protein and the additive.

It is preferable that the measurement data includes at least one of aggregation analysis data of a sub-visible particle of the protein in the candidate preservation solution, analysis data of the protein in the candidate preservation solution by a dynamic light scattering method, analysis data of the protein in the candidate preservation solution by size exclusion chromatography, or analysis data of the protein in the candidate preservation solution by differential scanning calorimetry.

It is preferable that the processor is configured to present auxiliary information corresponding to the score, the auxiliary information assisting in determination of whether or not the candidate preservation solution is adoptable.

It is preferable that the processor is configured to determine whether or not the score satisfies a selection condition set in advance and present a determination result as the auxiliary information.

It is preferable that the processor is configured to: output a plurality of the scores for each of a plurality of types of the candidate preservation solutions; determine a ranking of the candidate preservation solutions based on the plurality of scores; and present a determination result of the ranking as the auxiliary information.

It is preferable that the processor is configured to input the prescription information to the machine learning model in addition to the measurement data, the first feature amount, and the second feature amount.

It is preferable that the protein is an antibody.

It is preferable that the site is any of a fragment antigen-binding region, a fragment crystallizable region, a fragment variable region, various domains, or various complementarity determining regions.

According to the present disclosure, there is provided an operation method of a pharmaceutical support device, including: acquiring protein information related to a protein contained in a biopharmaceutical as a preservation target and including site information related to a plurality of sites constituting the protein, prescription information related to a prescription of a candidate preservation solution that is a candidate for a preservation solution for the biopharmaceutical as the preservation target, and measurement data indicating preservation stability of the candidate preservation solution measured by an actual test; deriving, based on the protein information and the prescription information, a first feature amount related to the preservation stability of the candidate preservation solution for an entire protein as a target and a second feature amount related to the preservation stability of the candidate preservation solution for each of the plurality of sites as a target; and inputting the measurement data, the first feature amount, and the second feature amount to a machine learning model and outputting a score indicating the preservation stability of the candidate preservation solution from the machine learning model.

According to the present disclosure, there is provided an operation program of a pharmaceutical support device causing a computer to execute a process including: acquiring protein information related to a protein contained in a biopharmaceutical as a preservation target and including site information related to a plurality of sites constituting the protein, prescription information related to a prescription of a candidate preservation solution that is a candidate for a preservation solution for the biopharmaceutical as the preservation target, and measurement data indicating preservation stability of the candidate preservation solution measured by an actual test; deriving, based on the protein information and the prescription information, a first feature amount related to the preservation stability of the candidate preservation solution for an entire protein as a target and a second feature amount related to the preservation stability of the candidate preservation solution for each of the plurality of sites as a target; and inputting the measurement data, the first feature amount, and the second feature amount to a machine learning model and outputting a score indicating the preservation stability of the candidate preservation solution from the machine learning model.

According to the technology of the present disclosure, it is possible to provide a pharmaceutical support device, an operation method of a pharmaceutical support device, and an operation program of a pharmaceutical support device that can improve prediction accuracy of preservation stability of a preservation solution.

### BRIEF DESCRIPTION OF THE DRAWINGS

Fig. 1 is a diagram illustrating a pharmaceutical support server and an operator terminal.
Fig. 2 is a diagram illustrating antibody information.
Fig. 3 is a diagram illustrating a series of flows of preparation of a candidate preservation solution, addition of an antibody, a stress test, and measurement of measurement data, prescription information, and the measurement data.
Fig. 4 is a diagram illustrating a basic structure of an antibody.
Fig. 5 is a block diagram illustrating a computer constituting the pharmaceutical support server.
Fig. 6 is a block diagram illustrating processing units of a CPU of the pharmaceutical support server.
Fig. 7 is a diagram illustrating a process of a feature amount derivation unit.
Fig. 8 is a diagram illustrating a process of a prediction unit.
Fig. 9 is a diagram illustrating processes of the prediction unit and a delivery control unit.
Fig. 10 is a diagram illustrating an information input screen.
Fig. 11 is a diagram illustrating an auxiliary information display screen.
Fig. 12 is a flowchart illustrating a processing procedure of the pharmaceutical support server.
Fig. 13 is a diagram illustrating a procedure according to the related art that selects a preservation solution suitable for a biopharmaceutical as a preservation target from a plurality of types of candidate preservation solutions.
Fig. 14 is a diagram illustrating a procedure according to the present example that selects a preservation solution suitable for a biopharmaceutical as a preservation target from a plurality of types of candidate preservation solutions.
Fig. 15 is a diagram illustrating another example of a second feature amount.
Fig. 16 is a diagram illustrating still another example of the second feature amount.
Fig. 17 is a diagram illustrating another example of a selection condition.
Fig. 18 is a diagram illustrating a process of a prediction unit according to a second embodiment.

### DESCRIPTION OF THE PREFERRED EMBODIMENTS

### [First Embodiment]

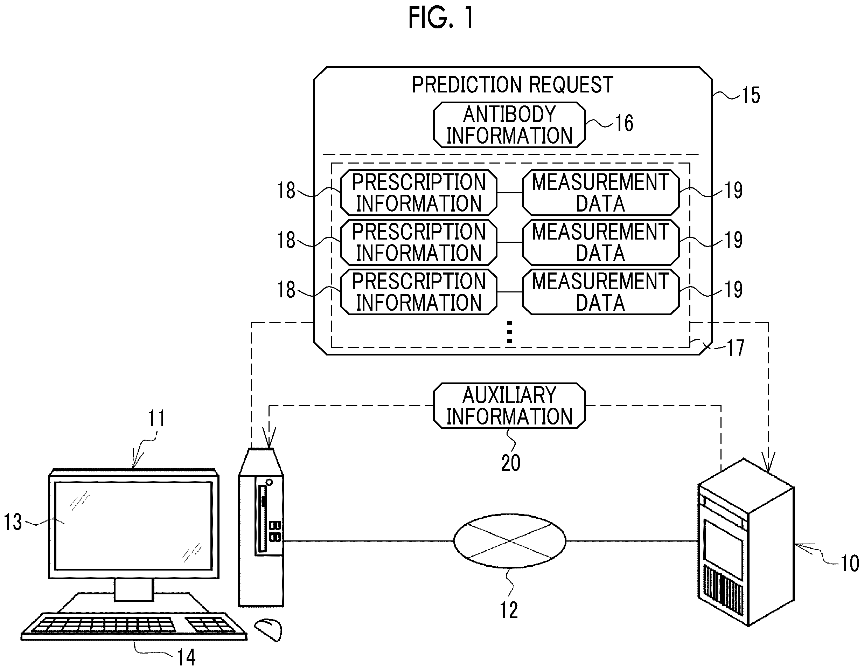

As illustrated in Fig. 1 as an example, a pharmaceutical support server 10 is connected to an operator terminal 11 through a network 12. The pharmaceutical support server 10 is an example of a "pharmaceutical support device" according to the technology of the present disclosure. The operator terminal 11 is installed, for example, in a pharmaceutical facility and is operated by an operator, such as a pharmacist, who is involved in the production of biopharmaceuticals in the pharmaceutical facility. The operator terminal 11 has a display 13 and an input device 14 such as a keyboard and a mouse. The network 12 is, for example, a wide area network (WAN) such as the Internet or a public communication network. In addition, in Fig. 1, only one operator terminal 11 is connected to the pharmaceutical support server 10. However, in practice, a plurality of operator terminals 11 in a plurality of pharmaceutical facilities are connected to the pharmaceutical support server 10.

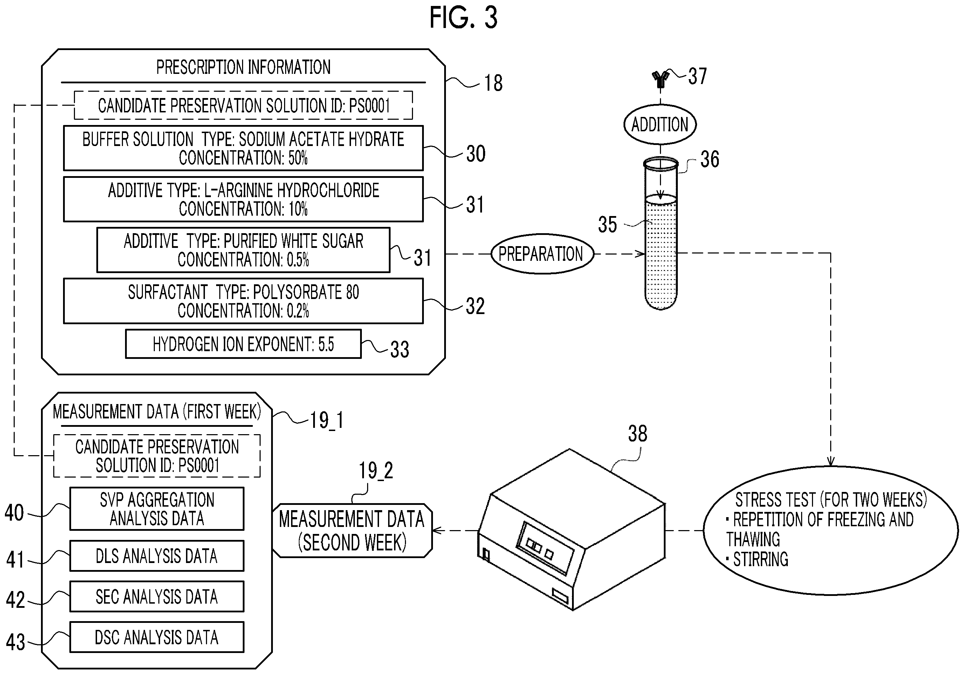

The operator terminal 11 transmits a prediction request 15 to the pharmaceutical support server 10. The prediction request 15 is a request for the pharmaceutical support server 10 to predict preservation stability of a candidate preservation solution 35 (see Fig. 3), which is a candidate for a preservation solution for the biopharmaceutical. The prediction request 15 includes antibody information 16 and a prescription information and measurement data set group 17. The antibody information 16 is information related to an antibody 37 (see Fig. 3) which is an active ingredient of the biopharmaceutical. The operator operates the input device 14 to input the antibody information 16. The antibody 37 is an example of a "protein" according to the technology of the present disclosure, and the antibody information 16 is an example of "protein information" according to the technology of the present disclosure.

The prescription information and measurement data set group 17 includes a plurality of sets of prescription information 18 and measurement data 19. The set of the prescription information 18 and the measurement data 19 is registered for each of a plurality of types, for example, several to several tens of types of candidate preservation solutions 35. The prescription information 18 is information related to the prescription of the candidate preservation solution 35. The measurement data 19 is data indicating the preservation stability of the candidate preservation solution 35 measured by an actual test. The operator also operates the input device 14 to input the prescription information 18 and the measurement data 19. In addition, although not illustrated, the prediction request 15 also includes terminal identification data (ID) for uniquely identifying the operator terminal 11 which is a transmission source of the prediction request 15.

In a case where the prediction request 15 is received, the pharmaceutical support server 10 derives auxiliary information 20 that assists in the determination of whether or not the candidate preservation solution 35 can be adopted. The pharmaceutical support server 10 delivers the auxiliary information 20 to the operator terminal 11 which is the transmission source of the prediction request 15. In a case where the auxiliary information 20 is received, the operator terminal 11 displays the auxiliary information 20 on the display 13 such that the auxiliary information 20 is provided to be viewed by the operator.

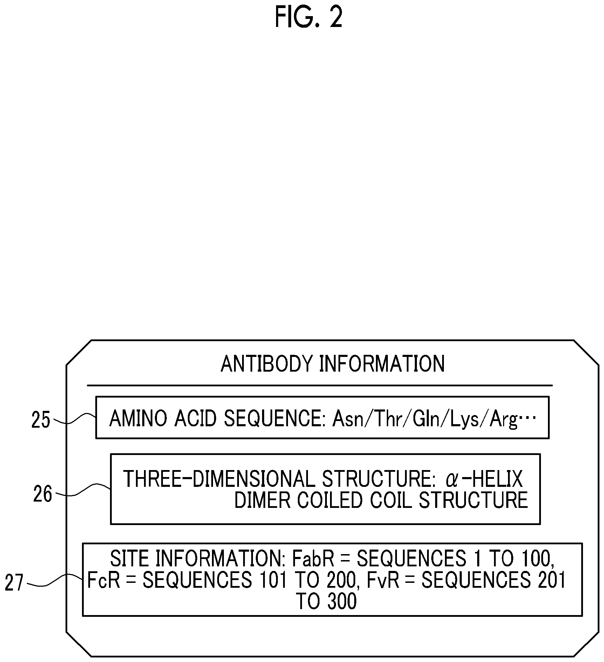

As illustrated in Fig. 2 as an example, the antibody information 16 includes an amino acid sequence 25 of the antibody 37, a three-dimensional structure 26 of the antibody 37, and site information 27. The antibody information 16 is obtained by analyzing the antibody 37 with a well-known technology such as mass spectrometry, X-ray crystal structure analysis, or electron microscopy. In the amino acid sequence 25, the order of peptide bonds of amino acids constituting the antibody 37, such as asparagine (abbreviated to ASn), glutamine (abbreviated to Glu), and arginine (abbreviated to Arg), is described from an amino terminal to a carboxyl terminal. The amino acid sequence 25 is also referred to as a primary structure. The three-dimensional structure 26 indicates a secondary structure, a tertiary structure, and a quaternary structure of the amino acids constituting the antibody 37. Examples of the secondary structure include a β-sheet, a β-turn, and the like in addition to α-helix given. Examples of the tertiary structure include a dimer coiled coil structure and the like. Examples of the quaternary structure include a dimer, a trimer, and a tetramer. Some antibodies 37 do not have the quaternary structure (that is, some antibodies 37 are monomers). In this case, the quaternary structure is not registered as described in the example. The three-dimensional structure 26 can also be obtained by inputting the amino acid sequence 25 to "AlphaFold2" which is a prediction program of a three-dimensional structure of the protein developed by DeepMind Technologies Limited.

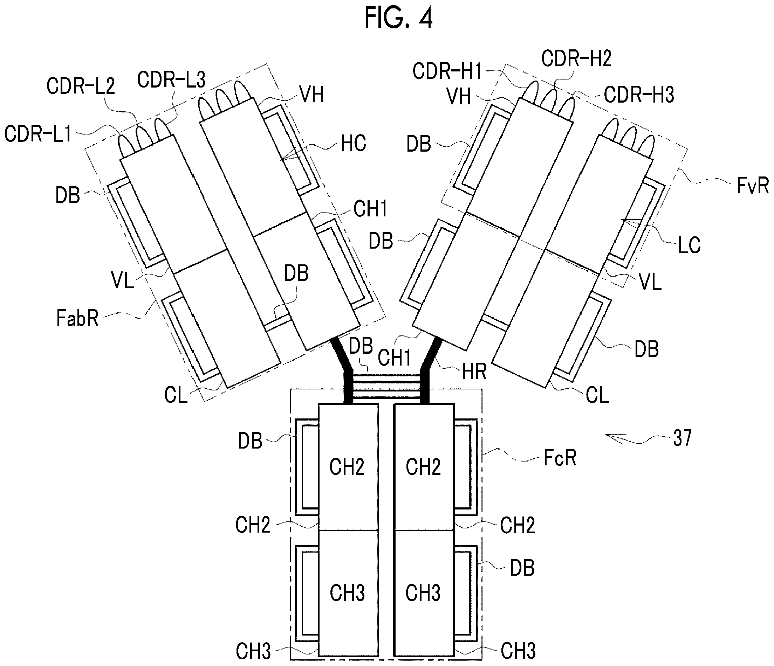

The site information 27 is information related to a plurality of sites constituting the antibody 37. Here, as the site information 27, information indicating which portion of the amino acid sequence 25 each site (all of which are illustrated in Fig. 4) of a fragment antigen-bindeig region FabR, a fragment crystallizable region FcR, and a fragment variable region FvR corresponds to is exemplified. The site information 27 is obtained by analyzing the antibody 37 by a well-known technology such as X-ray crystal structure analysis, or is also obtained from a database such as paper information or Protein Date Bank.

As illustrated in Fig. 3 as an example, the prescription information 18 includes a type and concentration 30 of a buffer solution, a type and concentration 31 of an additive, and a type and concentration 32 of a surfactant in the candidate preservation solution 35. The operator creates the prescription information 18, relying on the antibody information 16, his/her own experience, and the like. In some cases, each of a plurality of types of buffer solutions, additives, and surfactants is used as can be seen from L-arginine hydrochloride and purified white sugar which are the additives given as an example. In this case, a type and a concentration are registered for each of the plurality of types. Further, the prescription information 18 also includes a hydrogen ion exponent 33 of the candidate preservation solution 35. Furthermore, a molecular formula of each of the buffer solution, the additive, and the surfactant may be added to the prescription information 18.

The operator actually prepares the candidate preservation solution 35 in a test tube 36 in accordance with the prescription information 18. Then, the antibody 37 is added to the prepared candidate preservation solution 35. Then, a stress test including the repetition of freezing and thawing and stirring is performed for two weeks. During the stress test for two weeks, the measurement data 19 is actually measured using a measurement device 38. Fig. 3 illustrates an example in which measurement data 19_1 and measurement data 19_2 are measured in the first week and the second week of the stress test, respectively. The measurement device 38 transmits the measurement data 19 to the operator terminal 11.

The measurement data 19 includes aggregation analysis data (hereinafter, referred to as SVP aggregation analysis data) 40 of sub-visible particle (SVP) of the antibody 37 in the candidate preservation solution 35 and analysis data (hereinafter, referred to as DLS analysis data) 41 of the antibody 37 in the candidate preservation solution 35 by a dynamic light scattering (DLS) method as illustrated in the measurement data 19_1 in the first week. The SVP aggregation analysis data 40 indicates the amount of SVPs of the antibody 37 in the candidate preservation solution 35 that causes a reduction in the drug efficacy of the biopharmaceutical. Similarly, the DLS analysis data 41 indicates the amount of nanoparticles of the antibody 37 in the candidate preservation solution 35 that causes the reduction in the drug efficacy of the biopharmaceutical.

In addition, the measurement data 19 includes analysis data (hereinafter, referred to as SEC analysis data) 42 of the antibody 37 in the candidate preservation solution 35 by size exclusion chromatography (SEC) and analysis data (hereinafter, referred to as DSC analysis data) 43 of the antibody 37 in the candidate preservation solution 35 by differential scanning calorimetry (DSC). The SEC analysis data 42 indicates a molecular weight distribution of the antibody 37 in the candidate preservation solution 35. The DSC analysis data 43 indicates values related to thermophysical property values such as the glass transition point and crystallization temperature of the antibody 37 in the candidate preservation solution 35. In addition, in Fig. 3, for convenience, only one measurement device 38 is illustrated. However, in practice, various measurement devices 38 that measure these analysis data 40 to 43 are prepared.

A common candidate preservation solution ID is given to the prescription information 18 and the measurement data 19 of one candidate preservation solution 35. The prescription information 18 and the measurement data 19 are associated with each other by the candidate preservation solution ID. Further, in Fig. 3, only the preparation of one candidate preservation solution 35, the addition of the antibody 37, the stress test, and the measurement of the measurement data 19 are illustrated. However, in practice, the preparation, the addition, the stress test, and the measurement of the measurement data 19 are performed for each of a plurality of types of candidate preservation solutions 35.

As illustrated in Fig. 4 as an example, the antibody 37 basically has four polypeptide chains, that is, two identical heavy chains HC and two identical light chains LC. The antibody 37 has a configuration in which the two heavy chains HC and the two light chains LC are bonded by a disulfide bond DB, and has a Y shape that is bilaterally symmetrical.

The heavy chain HC is composed of a variable domain VH (variable domain, heavy chain) and constant domains CH (constant domain, heavy chain) 1, CH2, and CH3. The light chain LC is composed of a variable domain VL (variable domain, light chain) and a constant domain CL (constant domain, light chain). The constant domains CH1 and CH2 of the heavy chain HC are connected by a hinge region HR. The variable domains VH and VL are collectively referred to as a variable region. The constant domains CH1 to CH3 and CL are collectively referred to as a constant region.

The variable domain VH includes three complementarity determining regions CDR-H (complementarity determining region, heavy chain) 1, CDR-H2, and CDR-H3. In addition, the variable domain VL also includes three complementarity determining regions CDR-L (complementarity determining region, light chain) 1, CDR-L2, and CDR-L3. These complementarity determining regions CDR-H1 to CDR-H3 and CDR-L1 to CDR-L3 are bonding sites with an antigen and are also referred to as a hypervariable region.

A region composed of the variable domains VH and VL and the constant domains CH1 and CL is the fragment antigen-binding region FabR. A region composed of the constant domains CH2 and CH3 and a part of the hinge region HR is the fragment crystallizable region FcR. A region composed of the variable domains VH and VL is the fragment variable region FvR. As is well known, the fragment antigen-binding region FabR and the fragment crystallizable region FcR can be produced by papain digestion. Similarly, the fragment variable region FvR can be produced by pepsin digestion.

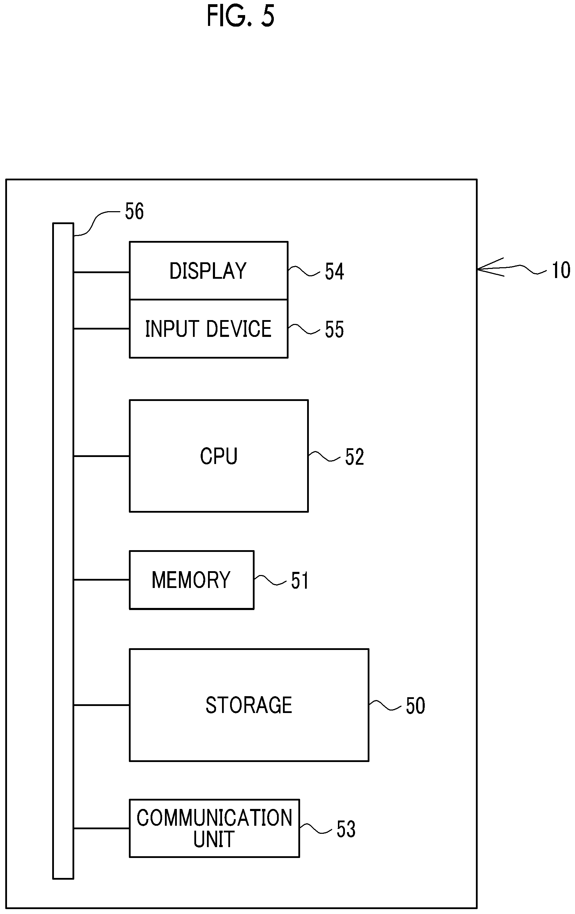

As illustrated in Fig. 5 as an example, a computer constituting the pharmaceutical support server 10 comprises a storage 50, a memory 51, a central processing unit (CPU) 52, a communication unit 53, a display 54, and an input device 55. These units are connected to each other through a bus line 56.

The storage 50 is a hard disk drive that is provided in the computer constituting the pharmaceutical support server 10 or that is connected to the computer through a cable or a network. Alternatively, the storage 50 is a disk array in which a plurality of hard disk drives are connected. The storage 50 stores, for example, a control program, such as an operating system, various application programs, and various types of data associated with these programs. In addition, a solid state drive may be used instead of the hard disk drive.

The memory 51 is a work memory used by the CPU 52 to execute processes. The CPU 52 loads the program stored in the storage 50 to the memory 51 and executes a process corresponding to the program. Therefore, the CPU 52 controls each unit of the computer comprehensively. The CPU 52 is an example of a "processor" according to the technology of the present disclosure. In addition, the memory 51 may be provided in the CPU 52.

The communication unit 53 controls the transmission of various types of information to an external device such as the operator terminal 11. The display 54 displays various screens. The various screens have operation functions by a graphical user interface (GUI). The computer constituting the pharmaceutical support server 10 receives an input of an operation instruction from the input device 55 through the various screens. The input device 55 is, for example, a keyboard, a mouse, a touch panel, and a microphone for voice input.

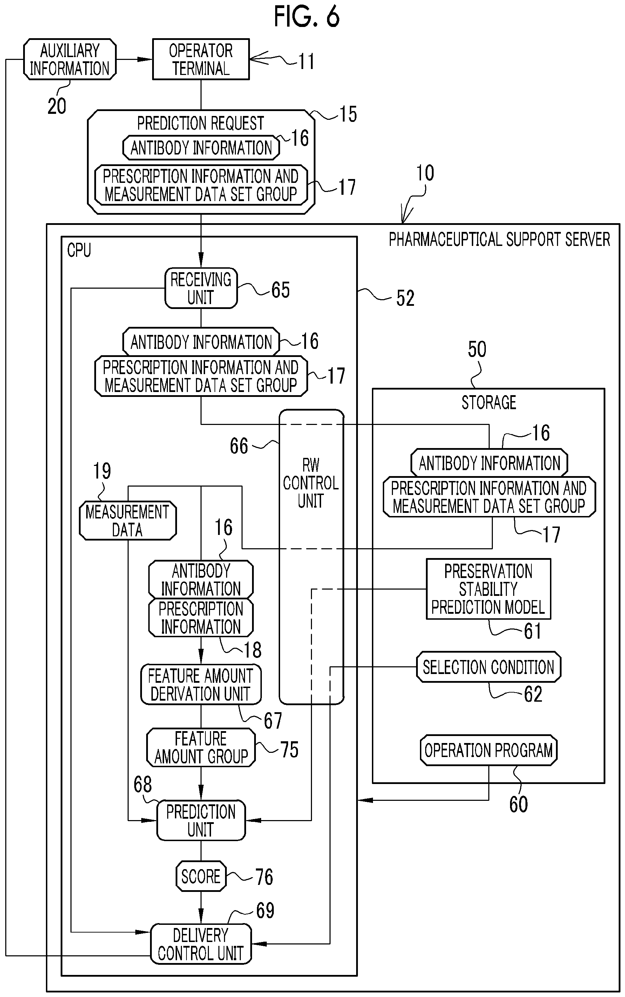

As illustrated in Fig. 6 as an example, an operation program 60 is stored in the storage 50 of the pharmaceutical support server 10. The operation program 60 is an application program for causing the computer to function as the pharmaceutical support server 10. That is, the operation program 60 is an example of "an operation program of a pharmaceutical support device" according to the technology of the present disclosure. The storage 50 also stores the antibody information 16 and the prescription information and measurement data set group 17. In addition, the storage 50 also stores a preservation stability prediction model 61 and a selection condition 62. The preservation stability prediction model 61 is an example of a "machine learning model" according to the technology of the present disclosure.

In a case where the operation program 60 is started, the CPU 52 of the computer constituting the pharmaceutical support server 10 functions as a receiving unit 65, a read and write (hereinafter, abbreviated to RW) control unit 66, a feature amount derivation unit 67, a prediction unit 68, and a delivery control unit 69 in cooperation with the memory 51 and the like.

The receiving unit 65 receives the prediction request 15 from the operator terminal 11. As described above, the prediction request 15 includes the antibody information 16 and the prescription information and measurement data set group 17, and the prescription information and measurement data set group 17 includes the prescription information 18 and the measurement data 19. Therefore, the receiving unit 65 acquires the antibody information 16, the prescription information 18, and the measurement data 19 by receiving the prediction request 15. The receiving unit 65 outputs the antibody information 16 and the prescription information and measurement data set group 17 to the RW control unit 66. In addition, the receiving unit 65 outputs the terminal ID of the operator terminal 11 (not illustrated) to the delivery control unit 69.

The RW control unit 66 controls the storage of various types of data in the storage 50 and the reading-out of various types of data in the storage 50. For example, the RW control unit 66 stores the antibody information 16 and the prescription information and measurement data set group 17 from the receiving unit 65 in the storage 50. Further, the RW control unit 66 reads out the antibody information 16 and the prescription information and measurement data set group 17 from the storage 50, outputs the antibody information 16 and the prescription information 18 to the feature amount derivation unit 67, and outputs the measurement data 19 to the prediction unit 68. Furthermore, the RW control unit 66 reads out the preservation stability prediction model 61 from the storage 50 and outputs the preservation stability prediction model 61 to the prediction unit 68. In addition, the RW control unit 66 reads out the selection condition 62 from the storage 50 and outputs the selection condition 62 to the delivery control unit 69.

The feature amount derivation unit 67 derives a feature amount group 75 that is a set of a plurality of feature amounts related to the preservation stability of the candidate preservation solution 35 based on the antibody information 16 and the prescription information 18. The feature amount derivation unit 67 outputs the derived feature amount group 75 to the prediction unit 68.

The prediction unit 68 derives a score 76 indicating the preservation stability of the candidate preservation solution 35 from the measurement data 19 and the feature amount group 75, using the preservation stability prediction model 61. The prediction unit 68 outputs the derived score 76 to the delivery control unit 69.

The delivery control unit 69 generates the auxiliary information 20 based on the score 76. The delivery control unit 69 controls the delivery of the auxiliary information 20 to the operator terminal 11 which is the transmission source of the prediction request 15. In this case, the delivery control unit 69 specifies the operator terminal 11, which is the transmission source of the prediction request 15, based on the terminal ID from the receiving unit 65.

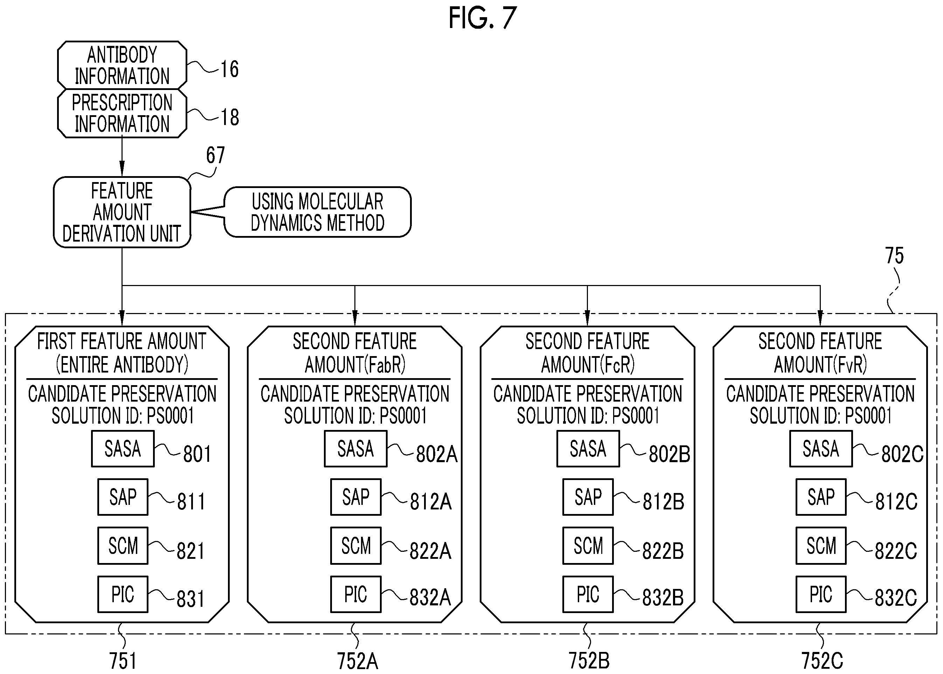

As illustrated in Fig. 7 as an example, the feature amount derivation unit 67 derives the feature amount group 75 using a molecular dynamics method. The feature amount group 75 is composed of a first feature amount 751 and second feature amounts 752A, 752B, and 752C. The first feature amount 751 is a feature amount related to the preservation stability of the candidate preservation solution 35 for the entire antibody 37 as a target. The second feature amount 752A is a feature amount related to the preservation stability of the candidate preservation solution 35 for the fragment antigen-binding region FabR as a target. The second feature amount 752B is a feature amount related to the preservation stability of the candidate preservation solution 35 for the fragment crystallizable region FcR as a target. The second feature amount 752C is a feature amount related to the preservation stability of the candidate preservation solution 35 for the fragment variable region FvR as a target. The feature amount derivation unit 67 derives the second feature amounts 752A to 752C with reference to the site information 27 of the antibody information 16. The fragment antigen-binding region FabR, the fragment crystallizable region FcR, and the fragment variable region FvR are examples of a "site" according to the technology of the present disclosure.

The first feature amount 751 includes a hydrophobic solvent accessible surface area (hereinafter, abbreviated to SASA) 801 of the entire antibody 37, a spatial aggregation propensity (hereinafter, abbreviated to SAP) 811 of the entire antibody 37, and a spatial charge map (hereinafter, abbreviated to SCM) 821 of the entire antibody 37. As the SASA 801 is larger and the SAP 811 is larger, the stability of the antibody 37 is lower. The SCM 821 indicates the degree of charge of the antibody 37. The SASA 801, the SAP 811, and the SCM 821 are derived based only on the antibody information 16 without referring to the prescription information 18. Therefore, the SASA 801, the SAP 811, and the SCM 821 are common to each candidate preservation solution 35.

In addition, the first feature amount 751 also includes a preferential interaction coefficient (hereinafter, abbreviated to PIC) 831 of the entire antibody 37 described in Non-Patent Document. The PIC 831 indicates the ease of bonding between the antibody 37 and the additive, more specifically, the degree to which the additive covers the surface of the antibody 37, and is an indicator indicating the compatibility between the antibody 37 and the additive. The PIC 831 makes it possible to know the possibility that the aggregation of the antibody 37 causing a reduction in the drug efficacy of the biopharmaceutical will occur. The PIC 831 is derived based on both the antibody information 16 and the prescription information 18 unlike the SASA 801, the SAP 811, and the SCM 821. Therefore, the PIC 831 is different in each candidate preservation solution 35. Therefore, a plurality of first feature amounts 751 of each candidate preservation solution 35 have the SASA 801, the SAP 811, and the SCM 821 in common and have different PICs 831. In addition, the candidate preservation solution ID is given to the first feature amount 751, as in the prescription information 18 and the measurement data 19. The candidate preservation solution ID is also given to the second feature amounts 752A to 752C.

The second feature amount 752A includes a hydrophobic SASA 802A of the fragment antigen-binding region FabR, an SAP 812A of the fragment antigen-binding region FabR, and an SCM 822A of the fragment antigen-binding region FabR. As the SASA 802A is larger and the SAP 812A is larger, the stability of the fragment antigen-binding region FabR is lower. The SCM 822A indicates the degree of charge of the fragment antigen-binding region FabR. Similarly to the case of the first feature amount 751, since the SASA 802A, the SAP 812A, and the SCM 822A are also derived based only on the antibody information 16, the SASA 802A, the SAP 812A, and the SCM 822A are common to each candidate preservation solution 35.

In addition, the second feature amount 752A also includes a PIC 832A of the fragment antigen-binding region FabR. The PIC 832A indicates the ease of bonding between the fragment antigen-binding region FabR and the additive, more specifically, the degree to which the additive covers the surface of the fragment antigen-binding region FabR, and is an indicator indicating the compatibility between the fragment antigen-binding region FabR and the additive. Similarly to the case of the first feature amount 751, since the PIC 832A is also derived based on both the antibody information 16 and the prescription information 18, the PIC 832A is different for each candidate preservation solution 35.

The second feature amount 752B includes a hydrophobic SASA 802B of the fragment crystallizable region FcR, an SAP 812B of the fragment crystallizable region FcR, and an SCM 822B of the fragment crystallizable region FcR. As the SASA 802B is larger and the SAP 812B is larger, the stability of the fragment crystallizable region FcR is lower. The SCM 822B indicates the degree of charge of the fragment crystallizable region FcR. Similarly to the case of the first feature amount 751 and the like, since the SASA 802B, the SAP 812B, and the SCM 822B are also derived based only on the antibody information 16, the SASA 802B, the SAP 812B, and the SCM 822B are common to each candidate preservation solution 35.

In addition, the second feature amount 752B also includes a PIC 832B of the fragment crystallizable region FcR. The PIC 832B indicates the ease of bonding between the fragment crystallizable region FcR and the additive, more specifically, the degree to which the additive covers the surface of the fragment crystallizable region FcR, and is an indicator indicating the compatibility between the fragment crystallizable region FcR and the additive. Similarly to the case of the first feature amount 751 and the like, since the PIC 832B is also derived based on both the antibody information 16 and the prescription information 18, the PIC 832B is different for each candidate preservation solution 35.

The second feature amount 752C includes a hydrophobic SASA 802C of the fragment variable region FvR, an SAP 812C of the fragment variable region FvR, and an SCM 822C of the fragment variable region FvR. As the SASA 802C is larger and the SAP 812C is larger, the stability of the fragment variable region FvR is lower. The SCM 822C indicates the degree of charge of the fragment variable region FvR. Similarly to the case of the first feature amount 751 and the like, since the SASA 802C, the SAP 812C, and the SCM 822C are also derived based only on the antibody information 16, the SASA 802C, the SAP 812C, and the SCM 822C are common to each candidate preservation solution 35.

In addition, the second feature amount 752C also includes a PIC 832C of the fragment variable region FvR. The PIC 832C indicates the ease of bonding between the fragment variable region FvR and the additive, more specifically, the degree to which the additive covers the surface of the fragment variable region FvR, and is an indicator indicating the compatibility between the fragment variable region FvR and the additive. Similarly to the case of the first feature amount 751 and the like, since the PIC 832C is also derived based on both the antibody information 16 and the prescription information 18, the PIC 832C is different for each candidate preservation solution 35. The PICs 831, 832A, 832B, and 832C may be derived using a machine learning model, such as a support vector machine (SVM), instead of the molecular dynamics method.

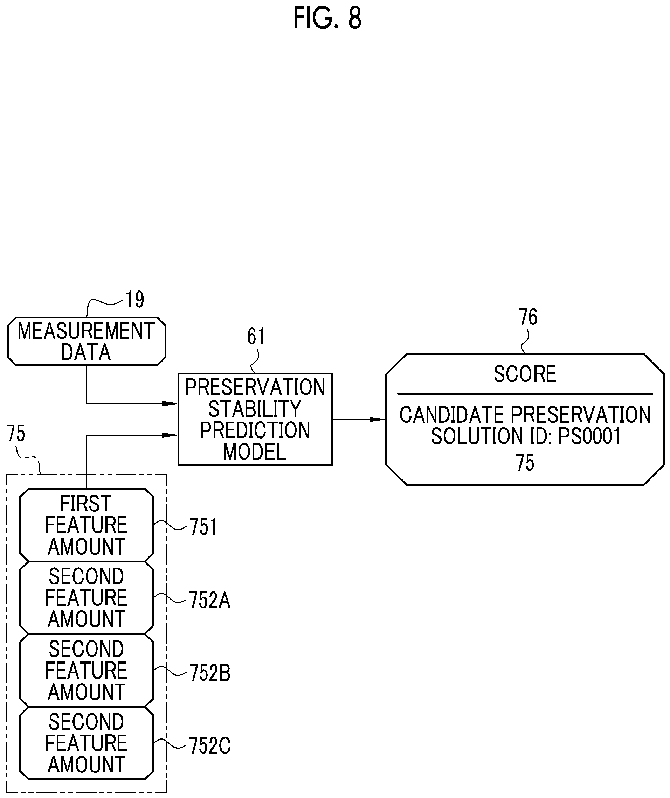

As illustrated in Fig. 8 as an example, the prediction unit 68 inputs the measurement data 19 and the feature amount group 75 (the first feature amount 751 and the second feature amounts 752A to 752C) to the preservation stability prediction model 61. Then, the score 76 is output from the preservation stability prediction model 61. The score 76 is represented by, for example, a continuous numerical value of 0 points to 100 points. The preservation stability prediction model 61 is a machine learning model using, for example, a support vector regression (SVR) algorithm that predicts the score 76 which is a continuous numerical value. The candidate preservation solution ID is given to the score 76, as in the first feature amount 751 and the like.

The learning of the preservation stability prediction model 61 may be performed in the pharmaceutical support server 10 or may be performed by a device other than the pharmaceutical support server 10. In addition, the learning of the preservation stability prediction model 61 may be continuously performed even after the operation.

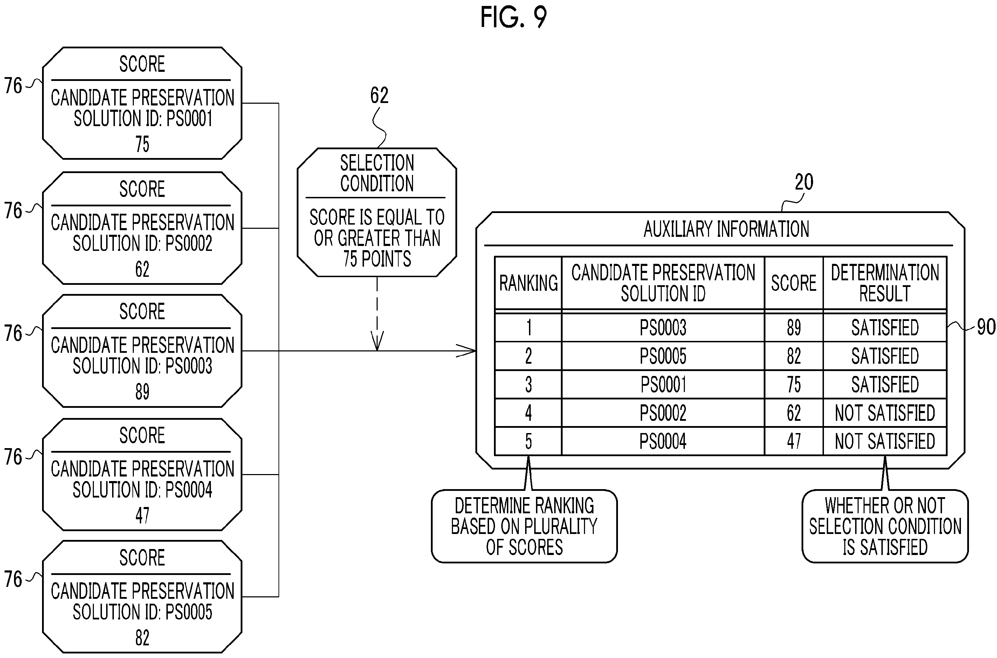

As illustrated in Fig. 9 as an example, the prediction unit 68 causes the preservation stability prediction model 61 to output a plurality of scores 76 for each of a plurality of types of candidate preservation solutions 35. The delivery control unit 69 determines ranking based on the plurality of scores 76 of the plurality of types of candidate preservation solutions 35 as shown in a table 90. In addition, the delivery control unit 69 determines whether or not the score 76 satisfies the selection condition 62. The delivery control unit 69 delivers the score 76, the determination result of the ranking based on the score 76, and the determination result of whether or not the score 76 satisfies the selection condition 62 as the auxiliary information 20 to the operator terminal 11.

Fig. 9 illustrates an example in which the ranking of five types of candidate preservation solutions 35 having candidate preservation solution IDs of PS0001 to PS0005 is determined and whether or not the score 76 satisfies the selection condition 62 is determined. The scores 76 of PS0001, PS0002, PS0003, PS0004, and PS0005 are 75 points, 62 points, 89 points, 47 points, and 82 points, respectively. For the ranking, PS0003 with the highest score of 89 points is in first place, PS0005 with the second highest score of 82 points is in second place, PS0001 with 75 points is in third place, PS0002 with 62 points is in fourth place, and PS0004 with the lowest score of 47 points is in fifth place. In addition, the content of the selection condition 62 is "the score is equal to or greater than 75 points". Therefore, the determination results for PS0003, PS0005, and PS0001 whose scores 76 are equal to or greater than 75 points are "satisfied", and the determination results for PS0002 and PS0004 whose scores 76 are less than 75 points are "not satisfied".

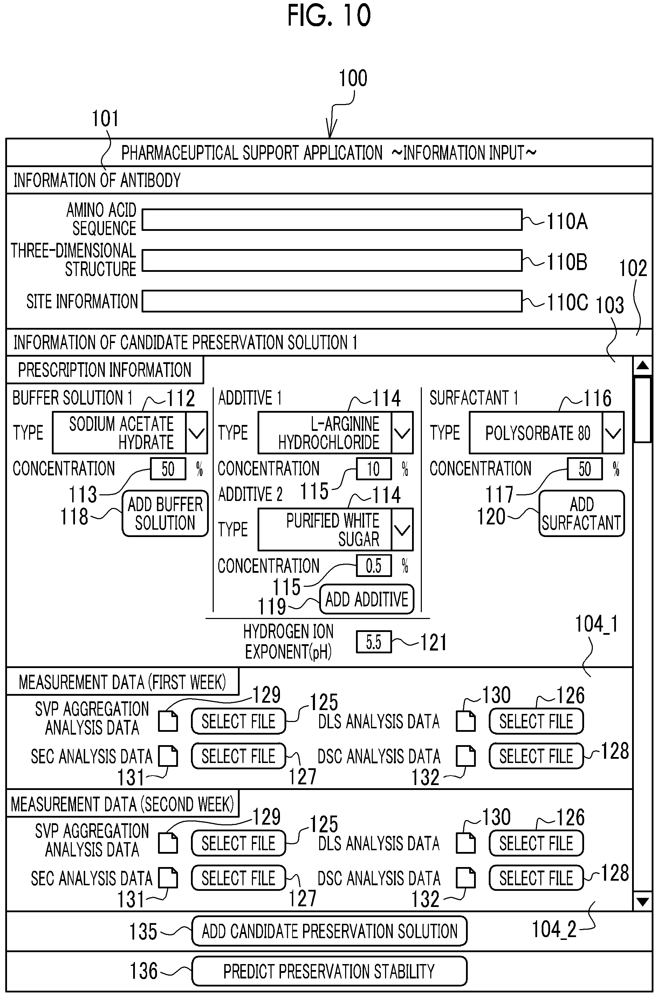

Fig. 10 illustrates an example of an information input screen 100 that is displayed on the display 13 of the operator terminal 11. The information input screen 100 has an input region 101 for the antibody information 16 and an input region 102 for the information of the candidate preservation solution 35. The input region 102 has an input region 103 for the prescription information 18, an input region 104_1 for the measurement data 19_1 in the first week, and an input region 104_2 for the measurement data 19_2 in the second week.

The input region 101 for the antibody information 16 is provided with an input box 110A for the amino acid sequence 25, an input box 110B for the three-dimensional structure 26, and an input box 110C for the site information 27.

The input region 103 for the prescription information 18 is provided with a pull-down menu 112 for selecting the type of the buffer solution, an input box 113 for the concentration of the buffer solution, a pull-down menu 114 for selecting the type of the additive, an input box 115 for the concentration of the additive, a pull-down menu 116 for selecting the type of the surfactant, and an input box 117 for the concentration of the surfactant.

An add button 118 for the buffer solution is provided below the pull-down menu 112 and the input box 113 for the buffer solution. In a case where the add button 118 is selected, the pull-down menu 112 and the input box 113 for the buffer solution are added. An add button 119 for the additive is provided below the pull-down menu 114 and the input box 115 for the additive. In a case where the add button 119 is selected, the pull-down menu 114 and the input box 115 for the additive are added. An add button 120 for the surfactant is provided below the pull-down menu 116 and the input box 117 for the surfactant. In a case where the add button 120 is selected, the pull-down menu 116 and the input box 117 for the surfactant are added. Further, an input box 121 for the hydrogen ion exponent is provided in a lower portion of the input region 103.

The input region 104 for the measurement data 19 is provided with a file selection button 125 for selecting a file of the SVP aggregation analysis data 40 and a file selection button 126 for selecting a file of the DLS analysis data 41. In addition, the input region 104 for the measurement data 19 is provided with a file selection button 127 for selecting a file of the SEC analysis data 42 and a file selection button 128 for selecting a file of the DSC analysis data 43.

In a case where the file of each of the analysis data 40 to 43 is selected, file icons 129, 130, 131, and 132 are displayed beside the file selection buttons 125 to 128, respectively. The file icons 129 to 132 are not displayed in a case where no files are selected.

An add button 135 for the candidate preservation solution 35 is provided in a lower portion of the input region 102 for the information of the candidate preservation solution 35. In a case where the add button 135 is selected, the input region 102 for the information of the candidate preservation solution 35 is added.

A prediction button 136 is provided below the add button 135 for the candidate preservation solution 35. In a case where the prediction button 136 is selected, the prediction request 15 including the antibody information 16 and the prescription information and measurement data set group 17 is transmitted from the operator terminal 11 to the pharmaceutical support server 10. The antibody information 16 includes contents input to the input boxes 110A to 110C. The prescription information and measurement data set group 17 includes contents selected by the pull-down menus 112, 114, and 116, contents input to the input boxes 113, 115, 117, and 121, and each of the analysis data 40 to 43 selected by the file selection buttons 125 to 128.

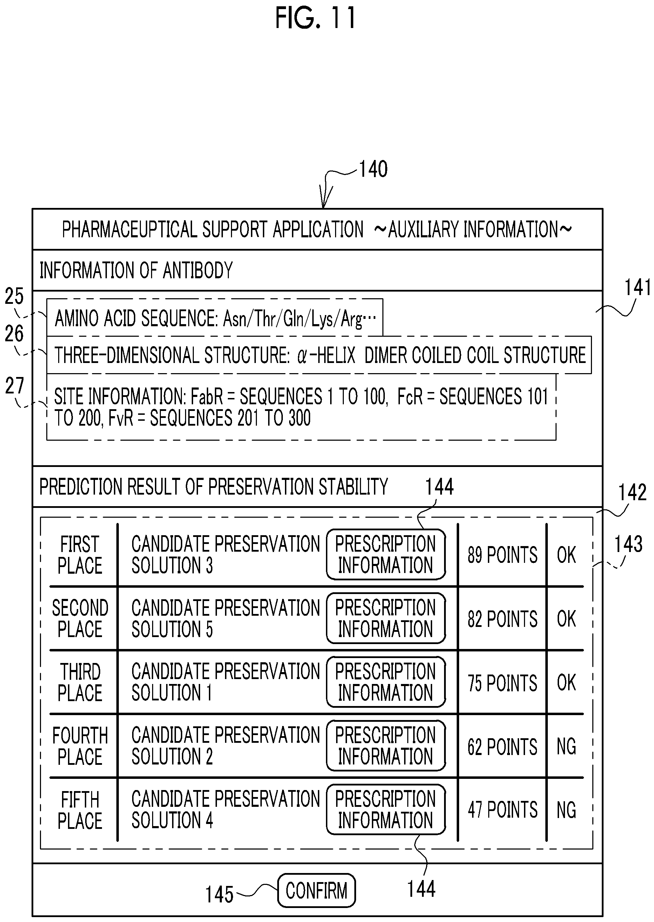

Fig. 11 illustrates an example of an auxiliary information display screen 140 that is displayed on the display 13 of the operator terminal 11 in a case where the auxiliary information 20 is received from the pharmaceutical support server 10. The auxiliary information display screen 140 has a display region 141 for the antibody information 16 and a display region 142 for the auxiliary information 20. The amino acid sequence 25, the three-dimensional structure 26, and the site information 27 are displayed in the display region 141 for the antibody information 16. A table 143 showing the ranking and score 76 of each candidate preservation solution 35 and the determination result of whether or not the score 76 satisfies the selection condition 62 is displayed in the display region 142 for the auxiliary information 20. In the table 143, "OK" is displayed as the determination result in a case where the score 76 satisfies the selection condition 62, and "NG" is displayed as the determination result in a case where the score 76 does not satisfy the selection condition 62. A display button 144 for the prescription information 18 is provided in the table 143. In a case where the display button 144 is selected, a display screen for the prescription information 18 of the candidate preservation solution 35 is displayed on the auxiliary information display screen 140 in a pop-up manner. The auxiliary information display screen 140 is removed by the selection of a confirm button 145.

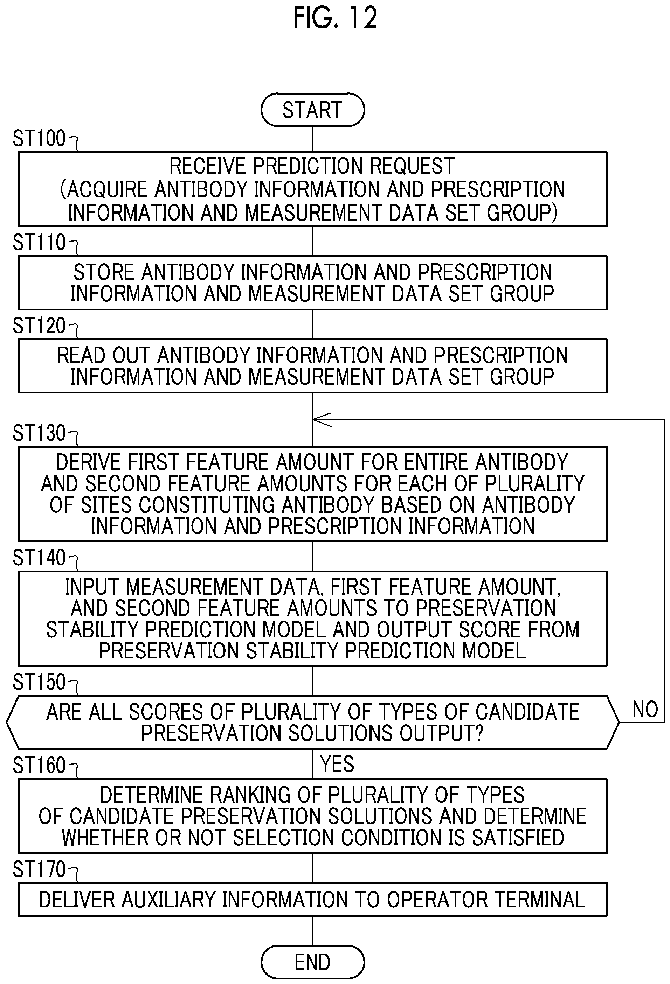

Next, the operation of the above-mentioned configuration will be described with reference to a flowchart illustrated in Fig. 12. First, in a case where the operation program 60 is started in the pharmaceutical support server 10, the CPU 52 of the pharmaceutical support server 10 functions as the receiving unit 65, the RW control unit 66, the feature amount derivation unit 67, the prediction unit 68, and the delivery control unit 69 as illustrated in Fig. 6.

The information input screen 100 illustrated in Fig. 10 is displayed on the display 13 of the operator terminal 11. The operator inputs the desired antibody information 16, prescription information 18, and measurement data 19 to the information input screen 100 and then selects the prediction button 136. Then, the prediction request 15 is transmitted from the operator terminal 11 to the pharmaceutical support server 10.

In the pharmaceutical support server 10, the prediction request 15 is received by the receiving unit 65. Therefore, the antibody information 16 and the prescription information and measurement data set group 17 (the prescription information 18 and the measurement data 19) are acquired (Step ST100). The antibody information 16 and the prescription information and measurement data set group 17 are output from the receiving unit 65 to the RW control unit 66 and are stored in the storage 50 under the control of the RW control unit 66 (Step ST110).

The RW control unit 66 reads out the antibody information 16 and the prescription information and measurement data set group 17 from the storage 50 (Step ST120). The antibody information 16 and the prescription information 18 are output from the RW control unit 66 to the feature amount derivation unit 67. The measurement data 19 is output from the RW control unit 66 to the prediction unit 68.

As illustrated in Fig. 7, the feature amount derivation unit 67 derives the feature amount group 75 based on the antibody information 16 and the prescription information 18 using the molecular dynamics method (Step ST130). The feature amount group 75 includes a first feature amount 751 related to the preservation stability of the candidate preservation solution 35 for the entire antibody 37 as a target. In addition, the feature amount group 75 includes the second feature amount 752A related to preservation stability of the candidate preservation solution 35 for the fragment antigen-binding region FabR as a target, a second feature amount 752B related to preservation stability of the candidate preservation solution 35 for the fragment crystallizable region FcR as a target, and a second feature amount 752C related to preservation stability of the candidate preservation solution 35 for the fragment variable region FvR as a target. The feature amount group 75 is output from the feature amount derivation unit 67 to the prediction unit 68.

Next, as illustrated in Fig. 8, in the prediction unit 68, the measurement data 19 and the feature amount group 75 (the first feature amount 751 and the second feature amounts 752A to 752C) are input to the preservation stability prediction model 61, and the score 76 is output from the preservation stability prediction model 61 (Step ST140). The score 76 is output from the prediction unit 68 to the delivery control unit 69. The processes in Steps ST130 and ST140 are repeatedly continued while all the scores 76 of the plurality of types of candidate preservation solutions 35 are not output (NO in Step ST150).

In a case where all the scores 76 of the plurality of types of candidate preservation solutions 35 are output (YES in Step ST150), the delivery control unit 69 determines the ranking of the candidate preservation solutions 35 based on each of a plurality of scores 76 of the plurality of types of candidate preservation solutions 35 as illustrated in Fig. 9. In addition, it is determined whether or not the score 76 satisfies the selection condition 62 (Step ST160). The determination result of the ranking and the determination result of whether or not the score 76 satisfies the selection condition 62 are delivered as the auxiliary information 20 to the operator terminal 11, which is the transmission source of the prediction request 15, under the control of the delivery control unit 69 (Step ST170).

The auxiliary information display screen 140 illustrated in Fig. 11 is displayed on the display 13 of the operator terminal 11. The operator views the auxiliary information display screen 140 and selects one candidate preservation solution 35 to be adopted as the preservation solution for the biopharmaceutical.

As described above, the CPU 52 of the pharmaceutical support server 10 comprises the receiving unit 65, the feature amount derivation unit 67, and the prediction unit 68. The receiving unit 65 acquires the antibody information 16, the prescription information 18, and the measurement data 19 by receiving the prediction request 15. The antibody information 16 is information related to the antibody 37 contained in the biopharmaceutical as a preservation target. The antibody information 16 includes a plurality of sites constituting the antibody 37, here, site information 27 related to the fragment antigen-binding region FabR, the fragment crystallizable region FcR, and the fragment variable region FvR. The prescription information 18 is information related to a prescription of the candidate preservation solution 35 which is a candidate for a preservation solution for a biopharmaceutical as a preservation target. The measurement data 19 is data indicating the preservation stability of the candidate preservation solution 35 measured by an actual test. The feature amount derivation unit 67 derives the first feature amount 751 related to the preservation stability of the candidate preservation solution 35 for the entire antibody 37 as a target based on the antibody information 16 and the prescription information 18. In addition, the feature amount derivation unit 67 derives the second feature amounts 752A to 752C related to the preservation stability of the candidate preservation solution 35 for each of a plurality of sites constituting the antibody 37, here, the fragment antigen-binding region FabR, the fragment crystallizable region FcR, and the fragment variable region FvR as a target. The prediction unit 68 inputs the measurement data 19, the first feature amount 751, and the second feature amounts 752A to 752C to the preservation stability prediction model 61, and outputs the score 76 indicating the preservation stability of the candidate preservation solution 35 from the preservation stability prediction model 61. The score 76 is output based on the measurement data 19 measured by the actual test and the second feature amounts 752A to 752C related to the preservation stability of the candidate preservation solution 35 for each of the plurality of sites constituting the antibody 37 as a target. Therefore, it is possible to improve the prediction accuracy of the preservation stability of the candidate preservation solution 35 as compared with a case where the measurement data 19 is not used and a case where only the first feature amount 751 for the entire antibody 37 is used as a target.

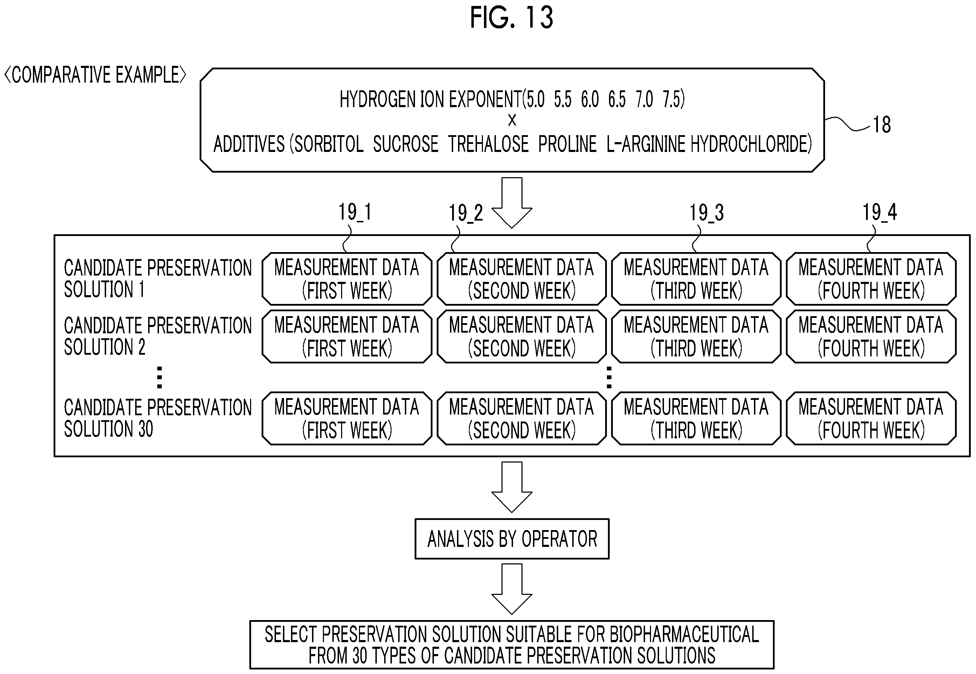

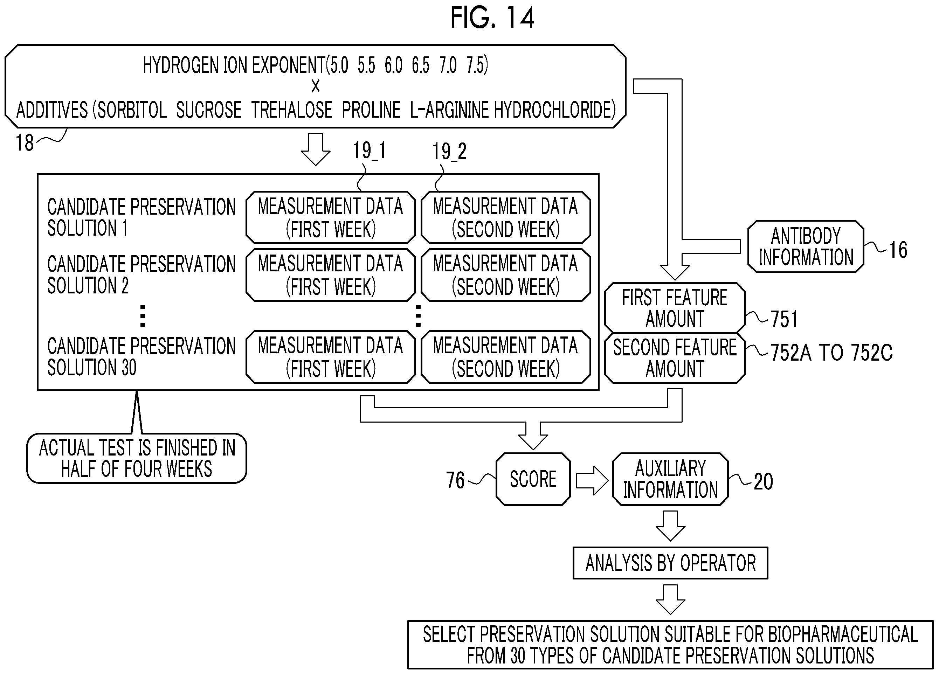

In addition, as illustrated in Figs. 13 and 14 as an example, it is also possible to reduce a complicated operation of preparing a plurality of types of candidate preservation solutions 35 and confirming the preservation stability of each candidate preservation solution 35 with the actual test.

Figs. 13 and 14 illustrate an example in which the hydrogen ion exponent is divided into 6 levels of 5.0, 5.5, 6.0, 6.5, 7.0, and 7.5 at an interval of 0.5, five types of additives of sorbitol, sucrose, trehalose, proline, and L-arginine hydrochloride is used as the additive, and one candidate preservation solution 35 to be adopted as the preservation solution for the biopharmaceutical is selected from 30 (= 6 × 5) types of candidate preservation solutions 35.

Fig. 13 illustrates a procedure according to the related art as a comparative example. In the related art, the stress test was performed on each of 30 types of candidate preservation solutions 35 for four weeks, and measurement data 19_1 in the first week, measurement data 19_2 in the second week, measurement data 19_3 in the third week, and measurement data 19_4 in the fourth week were actually measured. The operator analyzed the preservation stability of each candidate preservation solution 35 based on the measurement data 19 and selected one candidate preservation solution 35 to be adopted as the preservation solution for the biopharmaceutical from the 30 types of candidate preservation solutions 35.

In contrast, in the procedure according to the present example, as illustrated in Fig. 14, the operator finishes the stress test in two weeks that is half of four weeks. The score 76 indicating the preservation stability of the candidate preservation solution 35 is derived from the measurement data 19_1 in the first week and the measurement data 19_2 in the second week measured in the stress test for two weeks and the first feature amount 751 and the second feature amounts 752A to 752C derived from the antibody information 16 and the prescription information 18. Then, the auxiliary information 20 corresponding to the score 76 is presented to the operator. The measurement data 19_1 in the first week and the measurement data 19_2 in the second week are the results in the middle of the stress test for four weeks. The operator analyzes the preservation stability of each candidate preservation solution 35 based on the auxiliary information 20 and selects one candidate preservation solution 35 to be adopted as the preservation solution for the biopharmaceutical from the 30 types of candidate preservation solutions 35.

As described above, while it is necessary to perform the stress test for four weeks in the procedure according to the related art, the stress test can be completed in two weeks in the procedure according to the present example. Therefore, it is possible to reduce a complicated operation of preparing a plurality of types of candidate preservation solutions 35 and confirming the preservation stability of each candidate preservation solution 35 with the actual test.

Further, as the procedure according to the related art, there is a method that determines the prescription of the preservation solution step by step by, for example, narrowing down the type and concentration of the buffer solution in a first stage, narrowing down the type and concentration of the additive in a second stage, ..., narrowing down the numerical range of the hydrogen ion exponent in an N-th stage. According to the procedure of the present example, it is possible to reduce the operation in each stage. In addition, in some cases, the stage can be omitted. For example, the first stage is omitted, and the method starts from the second stage.

As illustrated in Fig. 2, the antibody information 16 includes the amino acid sequence 25 of the antibody 37 and the three-dimensional structure 26 of the antibody 37. Therefore, it is possible to derive the feature amount group 75 that more accurately indicates the preservation stability of the candidate preservation solution 35. In addition, antibody information 16 may include at least one of the amino acid sequence 25 of the antibody 37 or the three-dimensional structure 26 of the antibody 37.

As illustrated in Fig. 3, the prescription information 18 includes the types and concentrations 30 to 32 of each of the buffer solution, the additive, and the surfactant contained in the candidate preservation solution 35 and the hydrogen ion exponent 33 of the candidate preservation solution 35. Therefore, it is possible to derive the feature amount group 75 that more accurately indicates the preservation stability of the candidate preservation solution 35.

As illustrated in Fig. 7, the feature amount derivation unit 67 derives the feature amount group 75 using the molecular dynamics method. The molecular dynamics method is a computer simulation method that is popular in the field of biotechnology and can be introduced without any particular barriers. Therefore, it is possible to easily derive the feature amount group 75 without requiring a lot of time and effort.

As illustrated in Fig. 7, the first feature amount 751 includes the SASA 801, the SAP 811, and the SCM 821 of the entire antibody 37. In addition, the first feature amount 751 includes the PIC 831, which is an indicator indicating the compatibility between the entire antibody 37 and the additive contained in the candidate preservation solution 35. Similarly, the second feature amounts 752A to 752C include the SASAs 802A to 802C, the SAPs 812A to 812C, the SCMs 822A to 822C, and the PICs 832A to 832C of the fragment antigen-binding region FabR, the fragment crystallizable region FcR, and the fragment variable region FvR. Therefore, the score 76 that more accurately indicates the preservation stability of the candidate preservation solution 35 (the stability of each of the antibody 37 and the fragment antigen-binding region FabR, the fragment crystallizable region FcR, and the fragment variable region FvR) can be output from the preservation stability prediction model 61. The first feature amount 751 may include at least one of the SASA 801, the SAP 811, or the SCM 821 of the entire antibody 37. In addition, the second feature amount 752A may include at least one of the SASA 802A, the SAP 812A, or the SCM 822A. The same applies to the second feature amounts 752B and 752C.

As illustrated in Fig. 3, the measurement data 19 includes the SVP aggregation analysis data 40, the DLS analysis data 41, the SEC analysis data 42, and the DSC analysis data 43 of the antibody 37 in the candidate preservation solution 35. Therefore, the score 76 that more accurately indicates the preservation stability of the candidate preservation solution 35 can be output from the preservation stability prediction model 61. The measurement data 19 may include at least one of the SVP aggregation analysis data 40, the DLS analysis data 41, the SEC analysis data 42, or the DSC analysis data 43.

As illustrated in Fig. 9, the prediction unit 68 outputs a plurality of scores 76 for each of a plurality of types of candidate preservation solutions 35. The delivery control unit 69 presents the auxiliary information 20 corresponding to the score 76, the auxiliary information 20 assisting in the determination of whether or not the candidate preservation solution 35 can be adopted, by delivering the auxiliary information 20 to the operator terminal 11. More specifically, the delivery control unit 69 determines the ranking of the candidate preservation solution 35 based on the plurality of scores 76, and presents the determination result of the ranking as the auxiliary information 20. In addition, the delivery control unit 69 presents the determination result of whether or not the score 76 satisfies the selection condition 62 as the auxiliary information 20. Therefore, the operator selects the candidate preservation solution 35 to be adopted as the preservation solution for the biopharmaceutical.

The biopharmaceutical including the antibody 37 as the protein is called an antibody drug and is widely used not only for the treatment of chronic diseases, such as cancer, diabetes, and rheumatoid arthritis, but also for the treatment of rare diseases such as hemophilia and a Crohn's disease. Therefore, according to the present example in which the protein is the antibody 37, it is possible to further promote the development of antibody drugs widely used for the treatment of various diseases.

The site is the fragment antigen-binding region FabR, the fragment crystallizable region FcR, and the fragment variable region FvR. Therefore, the score 76 that more accurately indicates the preservation stability of the candidate preservation solution 35 can be output from the preservation stability prediction model 61.

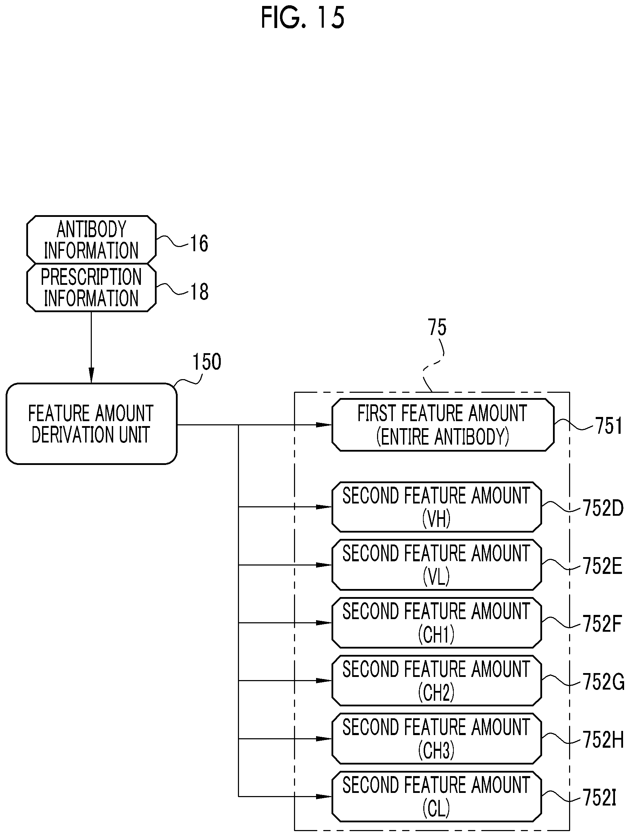

The site is not limited to the fragment antigen-binding region FabR, the fragment crystallizable region FcR, and the fragment variable region FvR given as an example. As illustrated in Fig. 15 as an example, the site may be various domains, that is, the variable domains VH and VL, and the constant domains CH1 to CH3 and CL. In this case, the feature amount derivation unit 150 derives the second feature amount 752D related to the preservation stability of the candidate preservation solution 35 for the variable domain VH as a target and the second feature amount 752E related to the preservation stability of the candidate preservation solution 35 for the variable domain VL as a target. In addition, the feature amount derivation unit 150 derives second feature amounts 752F, 752G, and 752H related to the preservation stability of the candidate preservation solution 35 for each of the constant domains CH1 to CH3 as a target and second feature amount 752I related to the preservation stability of the candidate preservation solution 35 for the constant domain CL as a target. The feature amount group 75 is composed of the first feature amount 751 and the second feature amounts 752D to 752I.

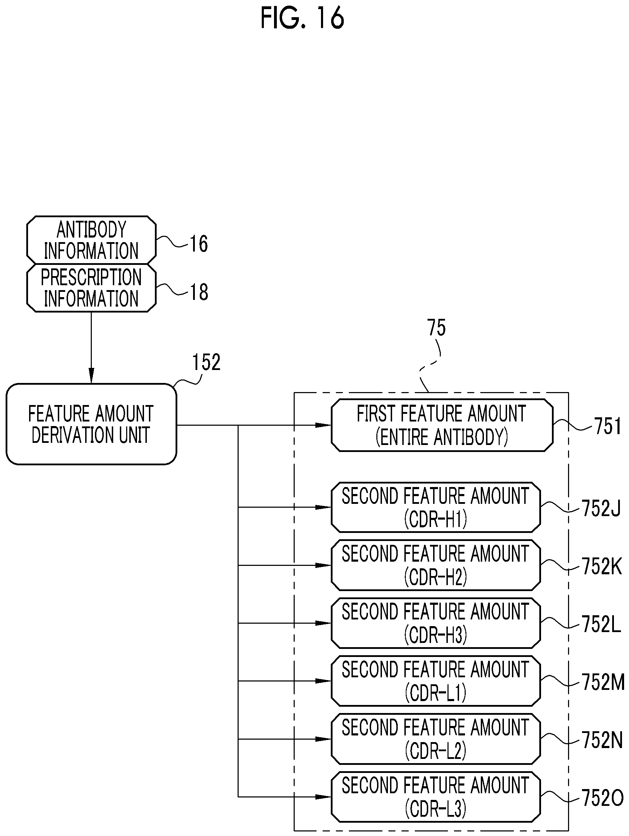

In addition, as illustrated in Fig. 16 as an example, the site may be various complementarity determining regions, that is, the complementarity determining regions CDR-H1 to CDR-H3 and CDR-L1 to CDR-L3. In this case, the feature amount derivation unit 152 derives second feature amounts 752J, 752K, and 752L related to the preservation stability of the candidate preservation solution 35 for each of the complementarity determining regions CDR-H1 to CDR-H3 as a target and second feature amounts 752M, 752N, and 752O related to the preservation stability of the candidate preservation solution 35 for each of the complementarity determining regions CDR-L1 to CDR-L3 as a target. The feature amount group 75 is composed of the first feature amount 751 and the second feature amounts 752J to 752O.

The site may be the fragment antigen-binding region FabR, the fragment crystallizable region FcR, and the fragment variable region FvR and various domains. In addition, the site may be the fragment antigen-binding region FabR, the fragment crystallizable region FcR, and the fragment variable region FvR and various complementarity determining regions. Furthermore, the site may be various domains and various complementarity determining regions. In short, the site may be any of the fragment antigen-binding region FabR, the fragment crystallizable region FcR, the fragment variable region FvR, various domains, or various complementarity determining regions.

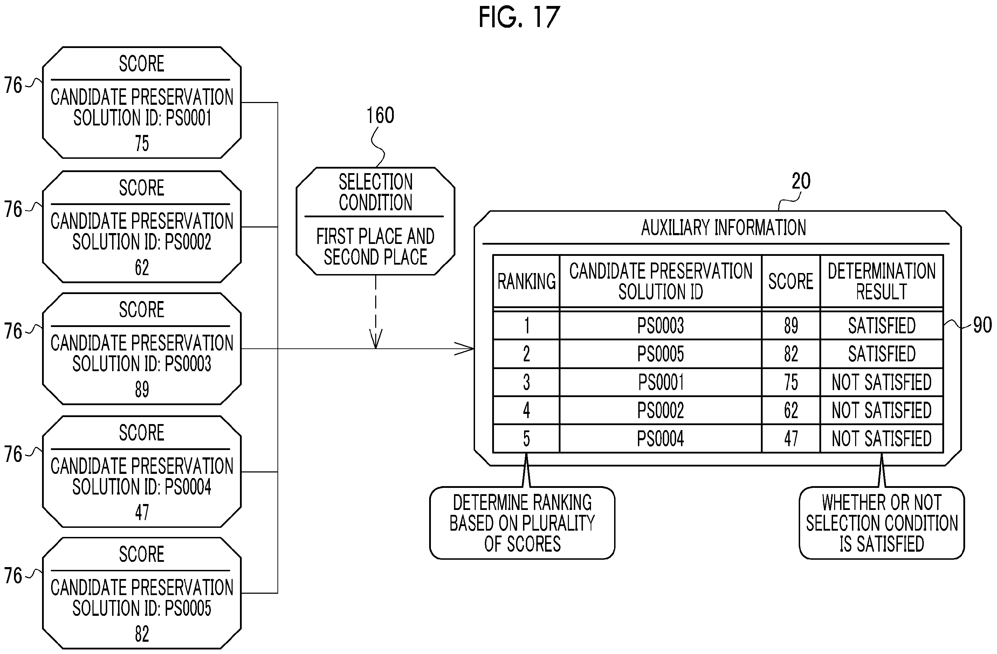

The selection condition is not limited to the content in which a threshold value is set for the score 76 like the selection condition 62 illustrated in Fig. 9 in which "the score is equal to or greater than 75 points". Like a selection condition 160 illustrated in Fig. 17 as an example, the content of the selection condition may be related to the ranking of the scores 76.

The content of the selection condition 160 is "first place and second place". Therefore, the determination results for PS0003 that is in first place of the ranking and PS0005 that is in second place of the ranking are "satisfied", and the determination results for PS0001, PS0002, and PS0004 that are in third to fifth places (not in first and second places) of the ranking are "not satisfied". In addition, the content of the selection condition related to the ranking may be, for example, "top 20%".

### [Second Embodiment]

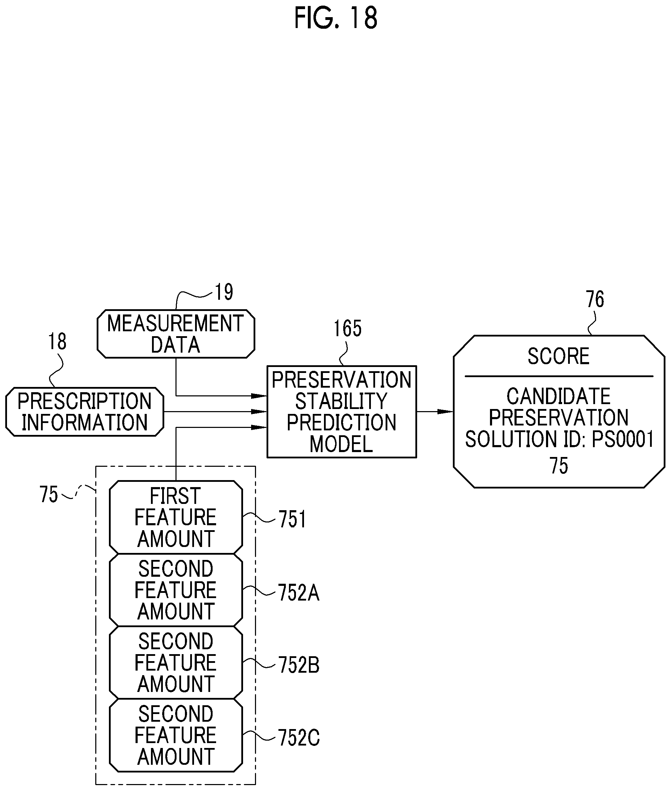

In a second embodiment illustrated in Fig. 18 as an example, in addition to the measurement data 19 and the feature amount group 75, the prescription information 18 is input to the preservation stability prediction model 165. For the types of the buffer solution, the additive, and the surfactant, numerical values set in advance for each type are input. Since the prescription information 18 is added to the input data of the preservation stability prediction model 165, it is possible to further improve the prediction accuracy of the score 76. The amino acid sequence 25 and the three-dimensional structure 26 in the antibody information 16 may be further input. Even in this case, for the amino acid sequence 25 and the three-dimensional structure 26, numerical values set in advance are input.

In each of the above-described embodiments, the description has been made on the premise that there are a plurality of types of candidate preservation solutions 35. However, the present disclosure is not limited thereto, and one type of candidate preservation solution 35 may be provided.

The protein is not limited to the antibody 37 given as an example. Examples of the protein may include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (a seventh factor, an eighth factor, a ninth factor, or the like), an enzyme (a lysosomal enzyme, a deoxyribonucleic acid (DNA) degrading enzyme, or the like), a fragment crystallizable (Fc) fusion protein, a receptor, albumin, and a protein vaccine. In addition, examples of the antibody include a bispecific antibody, an antibody-drug conjugate, a low-molecular-weight antibody, a sugar-chain-modified antibody, and the like.

Instead of delivering the auxiliary information 20 from the pharmaceutical support server 10 to the operator terminal 11, screen data of the auxiliary information display screen 140 illustrated in Fig. 11 may be delivered from the pharmaceutical support server 10 to the operator terminal 11.

The aspect in which the auxiliary information 20 is provided to be viewed by the operator is not limited to the auxiliary information display screen 140. A printed matter of the auxiliary information 20 may be provided to the operator, or an e-mail to which the auxiliary information 20 has been attached may be transmitted to a portable terminal of the operator.

The pharmaceutical support server 10 may be installed in each pharmaceutical facility or may be installed in a data center independent of the pharmaceutical facility. In addition, the operator terminal 11 may be configured to have some or all of the functions of each of the processing units 65 to 69 of the pharmaceutical support server 10.

In each of the above-described embodiments, for example, the following various processors can be used as a hardware structure of processing units executing various processes, such as the receiving unit 65, the RW control unit 66, the feature amount derivation units 67, 150, and 152, the prediction unit 68, and the delivery control unit 69. The various processors include, for example, the CPU 52 which is a general-purpose processor executing software (operation program 60) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to execute a specific process.

One processing unit may be configured by one of the various types of processors or may be configured by a combination of two or more processors of the same type or different types (for example, a combination of a plurality of FPGAs and/or a combination of a CPU and an FPGA). Further, a plurality of processing units may be configured by one processor.

As an example of configuring the plurality of processing units with one processor, first, there is an aspect in which one processor is configured by a combination of one or more CPUs and software and the processor functions as the plurality of processing units, as represented by computers such as a client and a server. A second example of the configuration is an aspect in which a processor that implements the functions of the entire system including a plurality of processing units using one integrated circuit (IC) chip is used. A representative example of this aspect is a system on chip (SoC). As described above, the various processing units are configured by using one or more of the above various processors as the hardware structure.

In addition, more specifically, an electric circuit (circuitry) in which circuit elements, such as semiconductor elements, are combined can be used as the hardware structure of the various processors.

It is possible to understand the technology described in the following supplementary notes from the above description.

### [Supplementary Note 1]

A pharmaceutical support device comprising:
a processor,
in which the processor is configured to:
   acquire protein information related to a protein contained in a biopharmaceutical as a preservation target and including site information related to a plurality of sites constituting the protein, prescription information related to a prescription of a candidate preservation solution that is a candidate for a preservation solution for the biopharmaceutical as the preservation target, and measurement data indicating preservation stability of the candidate preservation solution measured by an actual test;
   derive, based on the protein information and the prescription information, a first feature amount related to the preservation stability of the candidate preservation solution for an entire protein as a target and a second feature amount related to the preservation stability of the candidate preservation solution for each of the plurality of sites as a target; and
   input the measurement data, the first feature amount, and the second feature amount to a machine learning model and output a score indicating the preservation stability of the candidate preservation solution from the machine learning model.

### [Supplementary Note 2]

The pharmaceutical support device according to Supplementary Note 1,
in which the protein information includes at least one of an amino acid sequence of the protein or a three-dimensional structure of the protein.

### [Supplementary Note 3]

The pharmaceutical support device according to Supplementary Note 1 or 2,
in which the prescription information includes a type and concentration of each of a buffer solution, an additive, and a surfactant contained in the candidate preservation solution and a hydrogen ion exponent of the candidate preservation solution.

### [Supplementary Note 4]

The pharmaceutical support device according to any one of Supplementary Notes 1 to 3,
in which the processor is configured to derive the first feature amount and the second feature amount using a molecular dynamics method.

### [Supplementary Note 5]

The pharmaceutical support device according to Supplementary Note 4,
in which the first feature amount includes at least one of a solvent accessible surface area, a spatial aggregation propensity, or a spatial charge map of the entire protein, and
the second feature amount includes at least one of a solvent accessible surface area, a spatial aggregation propensity, or a spatial charge map of the sites constituting the protein.

### [Supplementary Note 6]

The pharmaceutical support device according to any one of Supplementary Notes 1 to 5,
in which the first feature amount includes an indicator indicating compatibility between the entire protein and an additive contained in the candidate preservation solution, and
the second feature amount includes an indicator indicating compatibility between the sites constituting the protein and the additive.

### [Supplementary Note 7]

The pharmaceutical support device according to any one of Supplementary Notes 1 to 6,
in which the measurement data includes at least one of aggregation analysis data of a sub-visible particle of the protein in the candidate preservation solution, analysis data of the protein in the candidate preservation solution by a dynamic light scattering method, analysis data of the protein in the candidate preservation solution by size exclusion chromatography, or analysis data of the protein in the candidate preservation solution by differential scanning calorimetry.

### [Supplementary Note 8]

The pharmaceutical support device according to any one of Supplementary Notes 1 to 7,
in which the processor is configured to present auxiliary information corresponding to the score, the auxiliary information assisting in determination of whether or not the candidate preservation solution is adoptable.

### [Supplementary Note 9]

The pharmaceutical support device according to Supplementary Note 8,
in which the processor is configured to:
determine whether or not the score satisfies a selection condition set in advance; and
present a determination result as the auxiliary information.

### [Supplementary Note 10]

The pharmaceutical support device according to Supplementary Note 8 or 9,
in which the processor is configured to:
output a plurality of the scores for each of a plurality of types of the candidate preservation solutions;
determine a ranking of the candidate preservation solutions based on the plurality of scores; and
present a determination result of the ranking as the auxiliary information.

### [Supplementary Note 11]

The pharmaceutical support device according to any one of Supplementary Notes 1 to 10,
in which the processor is configured to input the prescription information to the machine learning model in addition to the measurement data, the first feature amount, and the second feature amount.

### [Supplementary Note 12]

The pharmaceutical support device according to any one of Supplementary Notes 1 to 11,
in which the protein is an antibody.

### [Supplementary Note 13]

The pharmaceutical support device according to Supplementary Note 12,
in which the site is any of a fragment antigen-binding region, a fragment crystallizable region, a fragment variable region, various domains, or various complementarity determining regions.

The above various embodiments and/or various modification examples can be combined as appropriate in the technology of the present disclosure. In addition, it goes without saying that the present disclosure is not limited to each of the above-described embodiments, and various configurations can be adopted without departing from the gist of the present disclosure. Further, the technology of the present disclosure extends to a storage medium that stores the program non-transitorily, in addition to the program.

The description content and the illustrated content described above are detailed descriptions of portions according to the technology of the present disclosure and are merely an example of the technology of the present disclosure. For example, the above description of the configurations, functions, operations, and effects is the description of examples of the configurations, functions, operations, and effects of portions according to the technology of the present disclosure. Therefore, it is needless to say that unnecessary portions may be deleted or new elements may be added or replaced in the above description content and illustrated content without departing from the gist of the technology of the present disclosure. In order to avoid complication and facilitate understanding of the portion according to the technology of the present disclosure, the description related to common general knowledge not requiring special description in order to implement the technology of the present disclosure is omitted in the above description content and illustrated content.

In the present specification, "A and/or B" is synonymous with "at least one of A or B". That is, "A and/or B" may mean only A, only B, or a combination of A and B. Further, in the present specification, the same concept as "A and/or B" is also applied to a case where three or more matters are linked and expressed by "and/or".

All documents, patent applications, and technical standards described in the present specification are incorporated in the present specification by reference to the same extent as in a case where each of the documents, patent applications, and technical standards are specifically and individually indicated to be incorporated by reference.

## Claims

1. A pharmaceutical support device comprising:
a processor,
wherein the processor is configured to:
acquire protein information related to a protein contained in a biopharmaceutical as a preservation target and including site information related to a plurality of sites constituting the protein, prescription information related to a prescription of a candidate preservation solution that is a candidate for a preservation solution for the biopharmaceutical as the preservation target, and measurement data indicating preservation stability of the candidate preservation solution measured by an actual test;
derive, based on the protein information and the prescription information, a first feature amount related to the preservation stability of the candidate preservation solution for an entire protein as a target and a second feature amount related to the preservation stability of the candidate preservation solution for each of the plurality of sites as a target; and
input the measurement data, the first feature amount, and the second feature amount to a machine learning model and output a score indicating the preservation stability of the candidate preservation solution from the machine learning model.

2. The pharmaceutical support device according to claim 1,
wherein the protein information includes at least one of an amino acid sequence of the protein or a three-dimensional structure of the protein.

3. The pharmaceutical support device according to claim 1,
wherein the prescription information includes a type and concentration of each of a buffer solution, an additive, and a surfactant contained in the candidate preservation solution and a hydrogen ion exponent of the candidate preservation solution.

4. The pharmaceutical support device according to claim 1,
wherein the processor is configured to derive the first feature amount and the second feature amount using a molecular dynamics method.

5. The pharmaceutical support device according to claim 4,
wherein the first feature amount includes at least one of a solvent accessible surface area, a spatial aggregation propensity, or a spatial charge map of the entire protein, and
the second feature amount includes at least one of a solvent accessible surface area, a spatial aggregation propensity, or a spatial charge map of the sites constituting the protein.

6. The pharmaceutical support device according to claim 1,
wherein the first feature amount includes an indicator indicating compatibility between the entire protein and an additive contained in the candidate preservation solution, and
the second feature amount includes an indicator indicating compatibility between the sites constituting the protein and the additive.

7. The pharmaceutical support device according to claim 1,
wherein the measurement data includes at least one of aggregation analysis data of a sub-visible particle of the protein in the candidate preservation solution, analysis data of the protein in the candidate preservation solution by a dynamic light scattering method, analysis data of the protein in the candidate preservation solution by size exclusion chromatography, or analysis data of the protein in the candidate preservation solution by differential scanning calorimetry.

8. The pharmaceutical support device according to claim 1,
wherein the processor is configured to present auxiliary information corresponding to the score, the auxiliary information assisting in determination of whether or not the candidate preservation solution is adoptable.

9. The pharmaceutical support device according to claim 8,
wherein the processor is configured to:
determine whether or not the score satisfies a selection condition set in advance; and
present a determination result as the auxiliary information.

10. The pharmaceutical support device according to claim 8,
wherein the processor is configured to:
output a plurality of the scores for each of a plurality of types of the candidate preservation solutions;
determine a ranking of the candidate preservation solutions based on the plurality of scores; and
present a determination result of the ranking as the auxiliary information.

11. The pharmaceutical support device according to claim 1,
wherein the processor is configured to input the prescription information to the machine learning model in addition to the measurement data, the first feature amount, and the second feature amount.

12. The pharmaceutical support device according to claim 1,
wherein the protein is an antibody.

13. The pharmaceutical support device according to claim 12,
wherein the site is any of a fragment antigen-binding region, a fragment crystallizable region, a fragment variable region, various domains, or various complementarity determining regions.

14. An operation method of a pharmaceutical support device, comprising:
acquiring protein information related to a protein contained in a biopharmaceutical as a preservation target and including site information related to a plurality of sites constituting the protein, prescription information related to a prescription of a candidate preservation solution that is a candidate for a preservation solution for the biopharmaceutical as the preservation target, and measurement data indicating preservation stability of the candidate preservation solution measured by an actual test;
deriving, based on the protein information and the prescription information, a first feature amount related to the preservation stability of the candidate preservation solution for an entire protein as a target and a second feature amount related to the preservation stability of the candidate preservation solution for each of the plurality of sites as a target; and
inputting the measurement data, the first feature amount, and the second feature amount to a machine learning model and outputting a score indicating the preservation stability of the candidate preservation solution from the machine learning model.

15. An operation program of a pharmaceutical support device causing a computer to execute a process comprising:
acquiring protein information related to a protein contained in a biopharmaceutical as a preservation target and including site information related to a plurality of sites constituting the protein, prescription information related to a prescription of a candidate preservation solution that is a candidate for a preservation solution for the biopharmaceutical as the preservation target, and measurement data indicating preservation stability of the candidate preservation solution measured by an actual test;
deriving, based on the protein information and the prescription information, a first feature amount related to the preservation stability of the candidate preservation solution for an entire protein as a target and a second feature amount related to the preservation stability of the candidate preservation solution for each of the plurality of sites as a target; and
inputting the measurement data, the first feature amount, and the second feature amount to a machine learning model and outputting a score indicating the preservation stability of the candidate preservation solution from the machine learning model.
